(19) Europäisches Patentamt
European Patent Office
Office européen des brevets

(11) Veröffentlichungsnummer: **0 000 535**
**B1**

(12) # EUROPÄISCHE PATENTSCHRIFT

(45) Veröffentlichungstag der Patentschrift: **19.05.82**

(21) Anmeldenummer: **78100421.3**

(22) Anmeldetag: **18.07.78**

(51) Int. Cl.³: **C 07 D 303/08**, C 07 D 301/14

(54) **Verfahren zur Herstellung von halogensubstituierten Vinyl-Oxiranen.**

(30) Priorität: **29.07.77 DE 2734243**

(43) Veröffentlichungstag der Anmeldung:
**07.02.79 Patentblatt 79/3**

(45) Bekanntmachung des Hinweises auf die Patenterteilung:
**19.05.82 Patentblatt 82/20**

(84) Benannte Vertragsstaaten:
**BE CH DE FR GB NL**

(56) Entgegenhaltungen:
**FR - A - 2 300 085**
**FR - A - 2 369 273**
**FR - A - 2 369 274**

**JOURNAL OF ORGANIC CHEMISTRY, 41 (1976), Seiten 1648—1650**
***Seite 1648; Formel 5***

(73) Patentinhaber: **BAYER AG**
**Zentralbereich Patente, Marken und Lizenzen**
**D-5090 Leverkusen 1, Bayerwerk (DE)**

(72) Erfinder: **Rauleder, Gebhard, Dr.**
**Cheruskerstrasse 31**
**D-4000 Duesseldorf (DE)**
Erfinder: **Seifert, Hermann, Dr.**
**Ruwergasse 4**
**D-5000 Koeln 80 (DE)**
Erfinder: **Waldmann, Helmut, Dr.**
**Carl-Rumpff-Strasse 59**
**D-5090 Leverkusen 1 (DE)**
Erfinder: **Schwerdtel, Wulf, Dr.**
**Hegelstrasse 9**
**D-5090 Leverkusen 1 (DE)**
Erfinder: **Swodenk, Wolfgang, Dr.**
**Auf dem Broich 5**
**D-5068 Odenthal (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 000 535 B1

Verfahren zur Herstellung von halogensubstituierten Vinyl-Oxiranen

Die vorliegende Erfindung betrifft ein Verfahren zur Herstellung von halogensubstituierten Oxiranen aus halogensubstituierten Polyenen und Percarbonsäuren sowie neue 2-Chlor-2-vinyl- und 2-Chlor-3-vinyl-oxirane.

Am Oxiranring oder an der Vinylgruppe durch Halogen substituierte Vinyloxirane haben als Monomere aufgrund ihrer bifunktionellen Struktur eine besondere Bedeutung. Sie können deshalb auf dem Gebiet der lacke und Kunststoffe, der organischen Zwischenprodukte und im Pflanzenschutzbereich Verwendung finden, was besonders für die neuartigen bisher noch nicht hergestellten Verbindungen mit 2-Chlor-2-vinyl-oxiran- und 2-Chlor-3-vinyl-oxiran-Struktur von Interesse ist.

Zur Herstellung von (1-Halogenäthenyl-)oxiran sind bereits verschiedene Vorschläge gemacht worden. So berichtete Petrov 1939 über die Herstellung von (1-Chloräthenyl-)oxiran durch Umsetzen von Chloropren mit hypobromiger Säure und anschließender Dehydrobromierung mit Kaliumhydroxid. (A.A. Petrov, J. Gen. Chem. 9, 2232—43 (1939))

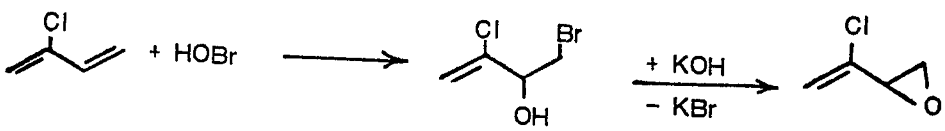


In zwei Patentschriften (USA—PS 2 907 774 und Brit.—PS 864 882) wurde 1957 ein Syntheseweg publiziert, bei dem (1-Chlor-äthenyl-)oxiran durch Dehydrochlorierung von (1,2-Dichloräthyl-)oxiran mit Alkylhydroxid synthetisiert wurde:

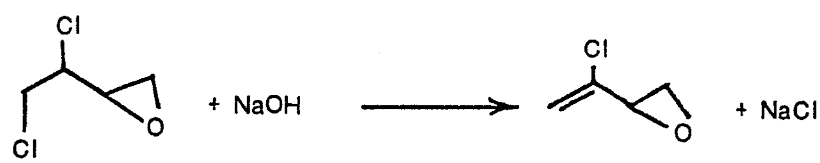


Nach diesen Methoden lassen sich (1-Halogenäthenyl-)oxirane synthetisieren; über ihre Anwendbarkeit zur Herstellung von am Oxiranring halogensubstituierten Vinyloxiranen ist jedoch noch nicht berichtet worden.

Weiterhin sind die französische Patentanmeldung mit der Veröffentlichungs-Nr. 2 300 085 und die Literaturstelle J. Org. Chem. *41*, Nr. 9, 1648 (1976) von Interesse.

Die französische Patentanmeldung betrifft die Umsetzung von Ethylen, Propylen, Chlor- oder Hydroxy-substituiertem Propylen, Buten, Penten und höheren Alkenen mit Percarbonsäure unter Bildung von Oxiranen. Angaben zur Qualität der eingesetzten Percarbonsäure werden nicht gemacht. Das einzige beschriebene Halogen enthaltende Einsatzprodukt ist Allylchlorid. Dieses enthält im Gegensatz zu den erfindungsgemäß einsetzbaren Verbindungen Halogen an einem gesättigten Kohlenstoffatom. Aus D. Swern, Organic Peroxides, Vol. II, Seite 490 ist bekannt, daß Epoxide, die am Epoxidring ein Chloratom enthalten, sich äußerst leicht in $\alpha$-Chlorketone umlagern und in vielen Fällen daß Epoxid nicht isoliert werden kann. Es hat deshalb nicht nahegelegen, die Umsetzung von Halogensubstituierten Polyenen mit Percarbonsäuren in Betracht zu ziehen, um Halogen-substituierte Vinyloxirane in guten Ausbeuten herzustellen bzw. das aus der französischen Patentanmeldung bekannte Verfahren auf Halogensubstituierte Polyene zu übertragen. Weiterhin geht aus Methodicum Chimicum, Georg Thieme Verlag, Stuttgart, Band 5, Seite 167/168 (1975) hervor, daß Halogen als Substituent die Epoxidation in starkem Maße erschwert und Folgereaktionen begünstigt. Insbesondere bei Vinylhalogeniden sind die Primärprodukte (Epoxide) häufig instabil und Umlagerungen in Carbonylverbindungen und Polymerisationen werden häufig beobachtet. Es ist auch im Hinblick auf diese Literaturstelle überraschend, daß es erfindungsgemäß gelingt, Verbindungen, die das Strukturelement "Vinylhalogenid" enthalten, in guten Ausbeuten zu epoxidieren.

Gemäß der Literaturstelle aus J. Org. Chem. wird angenommen, daß 4-Chlor-3,4-epoxy-3-methyl-1-buten als Zwischenprodukt in einer Reaktion auftreten könnte. Eine Möglichkeit, diese Verbindung herzustellen und zu isolieren, ist nicht beschrieben.

Demgegenüber wurde nun gefunden, daß man am Oxiranring und/oder an der Vinylgruppe halogensubstituierte Vinyloxirane aus halogensubstituierten Polyenen und Percarbonsäuren in organischer Lösung herstellen kann, wenn man ein halogensubstituiertes Polyen der Formel

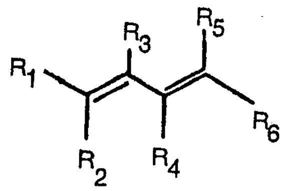

(I)

worin

$R_1$ und $R_6$ unabhängig voneinander Wasserstoff, $C_1$- bis $C_4$-Alkyl, Vinyl oder Phenyl bedeuten, $R_2$, $R_3$, $R_4$ und $R_5$ unabhängig voneinander für Wasserstoff, $C_1$- bis $C_4$-Alkyl, Vinyl, $C_3$- bis $C_7$-Cycloalkyl, Phenyl, Fluor, Chlor oder Brom stehen und wobei mindestens einer der Reste $R_2$ bis $R_5$ Fluor, Chlor oder Brom bedeutet und wobei $R_1$ mit $R_2$ oder $R_1$ mit $R_3$ oder $R_1$ mit $R_4$ oder $R_1$ mit $R_5$ oder $R_3$ mit $R_4$ einen carbocyclischen Ring bilden können, mit einer Lösung einer Percarbonsäure in einem organischen Lösungsmittel bei einem Molverhältnis von halogensubstituiertem Polyen zu Percarbonsäure von 1,0:1,0 bis 10, oder von 1,0 bis 10:1,0 und bei einer Temperatur von −20°C bis +100°C umsetzt, wobei die Percarbonsäure bis zu 5 Gew.-% Wasser, bis zu 2 Gew.-% Wasserstoffperoxid und die Percarbonsäurelösung weniger als 50 ppm Mineralsäure enthält.

Im Rahmen der Verbindungen der Formel (I) kommen beispielsweise Verbindungen der folgenden Formeln (II—V) besonders in Betracht:

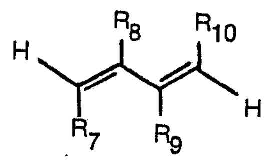

(II)

worin

$R_7$, $R_8$, $R_9$ und $R_{10}$ unabhängig voneinander Wasserstoff, $C_1$- bis $C_4$-Alkyl, Vinyl, $C_5$- bis $C_7$-Cycloalkyl, Phenyl, Fluor, Chlor oder Brom bedeuten, wobei mindestens einer der Reste $R_7$ bis $R_{10}$, Fluor, Chlor oder Brom darstellt und worin $R_7$ mit $R_8$ oder $R_8$ mit $R_9$ oder $R_9$ mit $R_{10}$ oder $R_8$ mit $R_9$ einen carbocyclischen Ring bilden können;

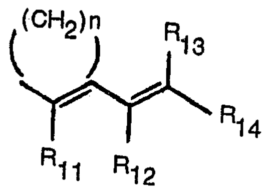

(III)

worin

$R_{11}$, $R_{12}$ und $R_{13}$ unabhängig voneinander für Wasserstoff, $C_1$- bis $C_4$-Alkyl, Vinyl, $C_5$- bis $C_7$-Cycloalkyl, Phenyl, Fluor, Chlor oder Brom stehen, wobei mindestens einer der Reste $R_{11}$ bis $R_{13}$ Fluor, Chlor oder Brom bedeutet, und

$R_{14}$ Wasserstoff, $C_1$- bis $C_4$-Alkyl, Vinyl oder Phenyl bedeutet,

n für eine ganze Zahl von 2 bis 10 steht;

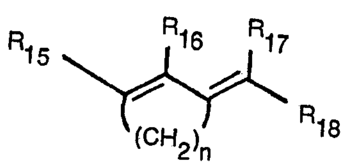

(IV)

worin

$R_{15}$, $R_{16}$ und $R_{17}$ unabhängig voneinander für Wasserstoff, $C_1$- bis $C_4$-Alkyl, Vinyl, $C_3$- bis $C_7$-Cycloalkyl, Phenyl, Fluor, Chlor oder Brom stehen, wobei mindestens einer der Reste $R_{15}$ bis $R_{17}$ Fluor, Chlor oder Brom bedeutet,

$R_{18}$ Wasserstoff, $C_1$- bis $C_4$-Alkyl, Vinyl oder Phenyl bedeutet und

n für eine ganze Zahl von 1 bis 9 steht;

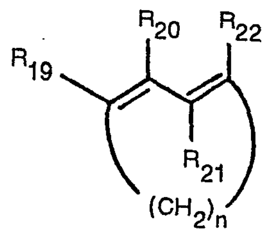

(V)

wobei

$R_{19}$, $R_{20}$, $R_{21}$ unabhängig voneinander Wasserstoff, $C_1$- bis $C_4$-Alkyl, Vinyl, Phenyl, $C_5$- bis $C_7$-Cycloalkyl, Fluor, Chlor oder Brom bedeuten, wobei mindestens einer der Reste $R_{19}$ bis $R_{22}$ für Fluor, Chlor oder Brom steht.

Besonders geeignet zur Umsetzung mit Percarbonsäuren gemäß dem erfindungsgemäßen Verfahren sind Polyene der Formel

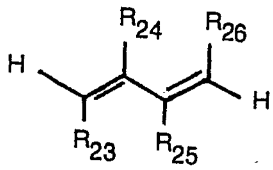

(VI)

worin

$R_{23}$, $R_{24}$, $R_{25}$ und $R_{26}$ unabhängig voneinander Wasserstoff, $C_1$- bis $C_4$-Alkyl, Vinyl oder Chlor bedeuten, wobei mindestens einer der Reste $R_{23}$ bis $R_{26}$ für Chlor steht.

Ganz besonders geeignet sind zur Umsetzung mit Percarbonsäuren nach dem erfindungsgemäßen Verfahren 1,3-Dichlorbutadien, 2,3-Dichlorbutadien, 1-Chlorbutadien und 2-Chlorbutadien.

Als organische Lösungsmittel können die verschiedenen Kohlenwasserstoffe verwendet werden, z.B aliphatische und cycloaliphatische Kohlenwasserstoffe wie Hexan, Heptan, Octan, 2-Äthyl-hexan, Decan, Dodecan, Cyclohexan, Methylcyclopentan, Petroläther; aromatische Kohlenwasserstoffe wie Benzol, Nitrobenzol, Toluol, Äthylbenzol, Cumol, Diisopropylbenzol, Xylol, Chlorbenzol; sauerstoffhaltige Kohlenwasserstoffe wie Diäthyläther, Diisopropyläther, Dibutyläther, Tetrahydrofuran, Dioxan, Pyran, Aceton, Methyläthylketon, Essigsäureäthylester, Essigsäuremethylester, Essigsäurepropylester, Essigsäurebutylester, Propionsäuremethylester, Propionsäure, äthylester, Propionsäurepropylester, Buttersäuremethylester, Buttersäureäthylester, Buttersäurepropylester, Buttersäurebutylester und chlorierte Kohlenwasserstoffe wie Methylenchlorid, Chloroform, Tetrachlorkohlenstoff, 1-Chloräthan, 1,2-Dichloräthan, 1,1-Dichloräthan, 1,1,2,2-Tetrachloräthan, 1-Chlorpropan, 2-Chlorpropan, 1,2-Dichlorpropan, 1,3-Dichlorpropan, 2,3-Dichlorpropan, 1,2,3-Trichlorpropan, 1,1,2,3-Tetrachlorpropan, Butylchlorid, 1,2-Dichlorbutan, 1,4-Dichlorbutan, 2,3-Dichlorbutan, 1,3-Dichlorbutan, 1,2,3,4-Tetrachlorbutan, tert.-Butylchlorid, Amylchlorid, 1,2-Dichlorpentan, 1,5-Dichlorpentan, 1,2,3,4-Tetrachlorpentan, Cyclopentylchlorid, 1,2-Dichlorcyclopentylchlorid, Hexylchlorid, 1,2-Dichlorhexan, 1,6-Dichlorhexan, 1,2,3,4-Tetrachlorhexan, 1,2,5,6-Tetrachlorhexan, Cyclohexylchlorid, 1,2-Dichlorhexan, Chlorbenzol, Heptylchlorid, 1,2-Dichlorheptan, 1,2,3,4-Tetrachlorheptan, Cycloheptylchlorid, 1,2-Dichlorheptan, Octylchlorid, 1,2-Dichloroctan, 1,2,3,4-Tetrachloroctan, Cyclooctylchlorid, und 1,2-Dichloroctan.

Bevorzugte Lösungsmittel sind von den chlorierten Kohlenwasserstoffen Methylchlorid, Chloroform, Tetrachlorkohlenstoff und 1,2-Dichlorpropan, von den aromatischen Kohlenwasserstoffen Benzol, Nitrobenzol, Toluol und Chlorbenzol, von den Kohlenwasserstoffen 2-Äthyl-hexan, Cyclohexan, Methyl-cyclopentan, von den sauerstoffhaltigen Kohlenwasserstoffen Aceton, Tetrahydrofuran, Propionsäureäthylester.

Besonders bevorzugte Lösungsmittel sind von den chlorierten Kohlenwasserstoffen 1,2-Dichlorpropan und Tetrachlorkohlenstoff, von den aromatischen Kohlenwasserstoffen Benzol und Chlorbenzol, von den Kohlenwasserstoffen Cyclohexan und von den sauerstoffhaltigen Kohlenwasserstoffen Propionsäureäthylester.

Verwendet werden können auch Lösungsmittelgemische der verschiedenen oben angegebenen organischen Lösungsmittel.

Erfindungsgemäß verwendbare Persäuren sind Perpropionsäure, Perbuttersäure und Perisobuttersäure. Bevorzugt verwendet werden Perpropionsäure und Perisobuttersäure. Besonders bevorzugt ist Perpropionsäure. Die Herstellung der mineralsäurefreien Persäuren in einem der genannten organischen Lösungsmittel kann z.B. nach dem in der DOS 2 262 970 beschriebenen Verfahren erfolgen.

Das erfindungsgemäße Verfahren wird in einem Temperaturbereich von –20 –100°C durchgeführt. Bevorzugt arbeitet man bei 0—60°C, besonders bevorzugt bei 10—40°C. In Sonderfällen können auch die angegebenen Temperaturen unter- oder überschritten werden.

Neben der Arbeitsweise unter isothermen Bedingungen, d.h. Einhaltung einer einheitlichen Temperatur im gesamten Reaktionsgemisch, kann man die Umsetzung auch unter Ausbildung eines sogenannten Temperaturgradienten durchführen, der im allgemeinen mit fortschreitender Reaktion zunimmt. Man kann aber auch die Reaktion so führen, daß sich mit dem Fortschreiten der Reaktion ein Gradient fallender Temperatur ausbildet.

Das Molverhältnis von halogensubstituierten Polyen zur Percarbonsäure beträgt erfindungsgemäß 1,0:1,0 bis 10 oder 1,0 bis 10:1,0. Angewendet werden kann auch ein Molverhältnis von Olefin zu Percarbonsäure von 1,0:1,0 bis 5 oder 1,0 bis 2:1,0. Bevorzugt wird ein Molverhältnis von 1,0:1 bis 5 angewendet. Ganz besonders vorteilhaft ist es, ein Molverhältnis von 2,0 bis 3,0 Mol halogensubstituiertes Polyen je Mol Persäure anzuwenden.

Das erfindungsgemäße Verfahren kann bei den verschiedensten Drücken durchgeführt werden. Im allgemeinen arbeitet man bei Normaldruck; das Verfahren kann jedoch auch bei Unter- oder Überdruck durchgeführt werden.

Der Wassergehalt der zur Epoxidation verwendeten Percarbonsäure beträgt biz zu 5 Gew.-%. Geeignet ist beispielsweise eine Percarbonsäure mit einem Wassergehalt von bis zu 1 Gew.-%. Vorzugsweise verwendet man eine Percarbonsäurelösung, die weniger als 0,5 Gew.-% Wasser enthält. Besonders bevorzugt ist ein Wassergehalt von weniger als 0,1 Gew.-%, z.B. 0,01 bis 0,1 Gew.-%.

Der Wasserstoffperoxidgehalt der angewendeten Percarbonsäure beträgt bis zu 2 Gew.-%. Vorteilhaft arbeitet man bei einem Gehalt von weniger als 1 Gew.-%. Besonders vorteilhaft ist es, die Umsetzung mit einer Percarbonsäurelösung durchzuführen, die einen Wasserstoffperoxidgehalt unterhalb 0,3% aufweist, z.B. 0,01 bis 0,3 Gew.-%.

Der Mineralsäuregehalt der zur Umsetzung gelangenden Percarbonsäurelösung beträgt weniger als 50 ppm. Besonders vorteilhaft ist ein Mineralsäuregehalt von weniger als 10 ppm.

Die Durchführung der Reaktion kann diskontinuierlich oder kontinuierlich in den für Umsetzungen dieser Art üblichen Vorrichtungen wie Rührwerkskessel, Siedereaktoren, Röhrenreaktoren, Schlaufenreaktoren oder Schleifenreaktoren erfolgen.

Schwermetallionen im Reaktionsgemisch katalysieren die Zersetzung der Percarbonsäure. Man setzt deshalb der Percarbonsäurelösung im allgemeinen Substanzen zu, die die Schwermetallionen durch Komplexbildung inaktivieren. Bekannte Substanzen solcher Art sind Gluconsäure, Äthylendiamintetraessigsäure, Natriumsilicat, Natriumpyrophosphat, Natriumhexametaphosphat, Dinatriumdimethylpyrophosphat oder $Na_2$ (2-äthylhexyl)$_5$($P_3O_{10}$)$_2$ (DAS 1 056 596, Spalte 4, Zeile 60 ff.).

Das halogensubstituierte Polyen kann auf verschiedene Art in die für die Umsetzung verwendete Vorrichtung eingebracht werden. Man kann es gemeinsam mit der Percarbonsäurelösung in den Reaktor geben oder man führt die beiden Komponenten getrennt voneinander dem Reaktor zu. Weiterhin ist es möglich, das Olefin und die Percarbonsäurelösung an verschiedenen Stellen in die Reaktoreinheit zu leiten. Bei Verwendung mehrerer in Kaskade geschalteter Reaktoren kann es zweckmäßig sein, das gesamte Olefin in den ersten Reaktor einzubringen. Man kann aber das Olefin auch auf die verschiedenen Reaktoren aufteilen.

Die Reaktionswärme wird durch innen- oder außenliegende Kühler abgeführt. Zur Ableitung der Reaktionswärme kann die Umsetzung auch unter Rückfluß, d.h. in Siedereaktoren, durchgeführt werden.

Die Reaktion wird zweckmäßigerweise unter möglichst vollständiger Umsetzung der Percarbonsäure vorgenommen. Im allgemeinen setzt man mehr als 95 Mol.-% der Percarbonsäure um. Zweckmäßig ist es, mehr als 98 Mol.-% Persäure umzusetzen.

Die Aufarbeitung des Reaktionsgemisches erfolgt in an sich bekannter Weise, z.B. durch Destillation. Bei einer bevorzugten Durchführung des Verfahrens wird eine etwa 20 Gew.-%ige Perpropionsäurelösung in Benzol unter Rühren zu der zweifachmolaren Menge halogensubstituiertem Polyen, das auf 30°C thermostatisiert ist, gegeben. Die Perpropionsäurelösung enthält weniger als 10 ppm Mineralsäure; sie hat einen Wassergehalt, der unterhalb von 0,1% liegt und weist einen Wasserstoffperoxidgehalt von weniger als 0,3% auf. Zur Komplexierung von Schwermetallionen wurde der Perpropionsäure vor der Umsetzung etwa 0,05 Gew.-% $Na_5$(2-Äthylhexyl)$_5$($P_3O_{10}$)$_2$ zugesetzt. Der Fortgang und das Ende der Reaktion werden kontrolliert, indem man der Reaktionslösung in zeitlichen Abständen Proben entnimmt und titrimetrisch den noch vorhandenen Gehalt an Percarbonsäure bestimmt. Nach Beendigung der Reaktion wird das Reaktionsgemisch fraktioniert.

Die folgenden Beispiele erläutern die Erfindung. Sämtliche Prozentangaben stellen, soweit nichts anderes gesagt wird, Gewichtsprozente dar.

## Beispiel 1

Umsetzung von Chloropren mit Perpropionsäure in Tetrachlorkohlenstoff.

Zu 88,54 g (1 Mol) Chloropren tropfte man bei 40°C unter Rühren innerhalb von 30 Minuten eine Lösung von 225 g (0,5 Mol) Perpropionsäure als 20·%ige Lösung in Tetrachlorkohlenstoff. Nach Zutropfende wurde noch weitere 4 Stunden bei dieser Temperatur gerührt, dann zeigte die titrimetrische Analyse einen Persäureumsatz von 98%. Die gaschromatographische Analyse zeigte, daß die beiden isomeren Oxirane 2-Chlor-2-vinyl-oxiran und (1-Chloräthenyl-)oxiran im Verhältnis von 3:2 gebildet wurden. Die Gesamtselektivät der beiden Oxirane betrug 83,5%, bezogen auf umgesetzte Perpropionsäure.

Beispiel 2

Umsetzung von Chloropren mit Perpropionsäure in Benzol. 88,54 g (1 Mol) Chloropren wurden, wie in Beispiel 1 beschrieben, mit 225 g (0,5 Mol) Perpropionsäure als 20 %ige Losung in Benzol bei 40°C umgesetzt.

Der Persäureumsatz betrug 99%. Die beiden Oxirane wurden wieder im Verhältnis von ~3:2 mit einer Gesamtelektivität von 84,5% gebildet.

Die Aufarbeitung des Reaktionsgemisches erfolgte destillativ. Zunächst wurde in einer Destillationskolonne, die mit einem Dünnschichtverdampfer bestückt war, Chloropren, Benzol und die beiden Oxirane als Kopfprodukt entnommen, während am Sumpf Propionsäure erhalten wurde. Durch weitere Fraktionierung bei vermindertem Druck wurden die beiden Oxirane, 2-Chlor-2-vinyl-oxiran und (1-Chloräthenyl-)oxiran in einer Reinheit von über 99% isoliert.

Beispiel 3

Umsetzung von 2,3-Dichlor-1,3-butadien mit Perpropionsäure in Benzol. 123 g (1 Mol) 2,3-Dichlor-1,3-butadien wurden bei 30°C mit 225 g (0,5 Mol) Perpropionsäure als 20%ige Lösung in Benzol wie in Beispiel 1 beschrieben umgesetzt. Nach drei Stunden Reaktionszeit betrug der Persäureumsatz 98,7%. Die GC-Analyse zeigte, daß 2-Chlor-(1-chloräthenyl-)-oxiran mit einer Selektivität von 78% gebildet wurde.

Beispiel 4

Umsetzung von 1-Chlor-1,3-Butadien mit Perpropionsäure in Benzol. 88,54 g (1 Mol) 1-Chlor-1,3-butadien wurden bei 40°C wie in Beispiel 1 beschrieben mit einer Lösung von 225 g (0,5 Mol) Perpropionsäure als 20%ige Lösung in Benzol umgesetzt. Nach 4 Stunden Reaktionsdauer betrug der Persäureumsatz 99%. Die beiden Oxirane 2-Chlor-3-vinyl-oxiran und (2-Chloräthenyl-)oxiran wurden mit einer Gesamtselektivität von 87% gebildet.

Beispiel 5

Umsetzung von 1,4-Dichlor-1,3-butadien mit Perpropionsäure in 1,2-Dichlorpropan.

123 g (1 Mol) 1,4-Dichlor-1,3-butadien wurden bei 30°C mit 225 g (0,5 Mol) Perpropionsäure als 20%ige Lösung in Dichlorpropan wie in Beispiel 1 beschrieben umgesetzt.

Nach 4 Stunden Reaktionszeit betrug der Persäureumsatz 99%. 2-Chlor-3-(2-chloräthenyl-)oxiran wurde mit einer Selektivität von 81% gebildet.

**Patentansprüche**

1. Verfahren zur Herstellung von am Oxiranring und/oder an der Vinylgruppe halogensubstituierten Vinyloxiranen aus halogensubstituierten Polyenen und Percarbonsäuren in organischer Lösung, dadurch gekennzeichnet, daß man ein halogensubstituiertes Polyen der Formel

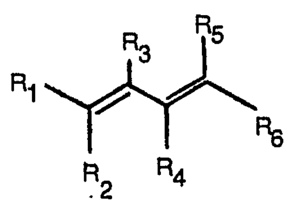

worin

$R_1$ und $R_6$ unabhängig voneinander Wasserstoff, $C_1$- bis $C_4$-Alkyl, Vinyl oder Phenyl bedeuten, $R_2$, $R_3$, $R_4$ und $R_5$ unabhängig voneinander für Wasserstoff, $C_1$- bis $C_4$-Alkyl, Vinyl, $C_3$- bis $C_7$-Cycloalkyl, Phenyl, Fluor, Chlor oder Brom stehen und wobei mindestens einer der Reste $R_2$ bis $R_5$ Fluor, Chlor oder Brom bedeutet und wobei $R_1$ mit $R_2$ oder $R_1$ mit $R_3$ oder $R_1$ mit $R_4$ oder $R_1$ mit $R_5$ oder $R_3$ mit $R_4$ einen carbocyclischen Ring bilden können,

mit einer Lösung einer Percarbonsäure in einem organischen Lösungsmittel bei einem Molverhältnis von halogensubstituiertem Polyen zu Percarbonsäure von 1,0:1,0 bis 10 oder 1,0 bis 10:1,0 und bei einer Temperatur von –20°C bis +100°C umsetzt, wobei die Percarbonsäure bis zu 5 Gew.-% Wasser, bis 2 Gew.-% Wasserstoffperoxid und die Percarbonsäurelösung weniger als 50 ppm Mineralsäure enthält.

2. Verfahren nach Anspruch 1, dadurch gekennzeichnet, daß man als halogensubstituiertes Polyen ein Polyen der Formel

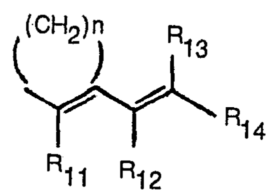

worin

$R_{11}$, $R_{12}$ und $R_{13}$ unabhängig voneinander für Wasserstoff, $C_1$- bis $C_4$-Alkyl, Vinyl, $C_5$- bis $C_7$ Cycloalkyl, Phenyl, Fluor, Chlor oder Brom stehen, wobei mindestens einer der Reste $R_{11}$ bis $R_{13}$ Fluor, Chlor oder Brom bedeutet, und

$R_{14}$ Wasserstoff, $C_1$- bis $C_4$-Alkyl, Vinyl, Phenyl bedeuten,

n für eine ganze Zahl von 2 bis 10 steht,

einsetzt.

3. Verfahren nach Anspruch 1 und 2, dadurch gekennzeichnet, daß man als halogensubstituiertes Polyen ein Polyen der Formel

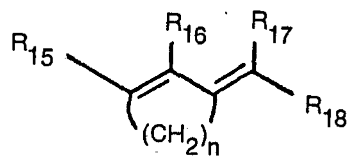

worin

$R_{15}$, $R_{16}$ und $R_{17}$ unabhängig voneinander für Wasserstoff, $C_1$- bis $C_4$-Alkyl, Vinyl, $C_3$- bis $C_7$-Cycloalkyl, Phenyl, Fluor, Chlor oder Brom stehen, wobei mindestens einer der Reste $R_{15}$ bis $R_{17}$ Fluor, Chlor oder Brom bedeutet, und

$R_{18}$ Wasserstoff, $C_1$- bis $C_4$-Alkyl, Vinyl, Phenyl bedeuten, und

n für eine ganze Zahl von 1 bis 9 steht,

einsetzt.

4. Verfahren nach Anspruch 1 bis 3, dadurch gekennzeichnet, daß daß man als halogensubstituiertes Polyen ein Polyen der Formel

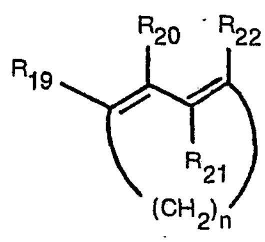

worin

$R_{19}$, $R_{20}$, $R_{21}$ und $R_{22}$ unabhängig voneinander Wasserstoff, $C_1$- bis $C_4$-Alkyl, Vinyl, Phenyl, $C_5$- bis $C_7$-Cycloalkyl, Fluor, Chlor oder Brom bedeuten, wobei mindestens einer der Reste $R_{19}$ bis $R_{22}$ für Fluor, Chlor oder Brom steht,

einsetzt.

5. Verfahren nach Anspruch 1 bis 4, dadurch gekennzeichnet, daß man als halogensubstituiertes Polyen ein Polyen der Formel

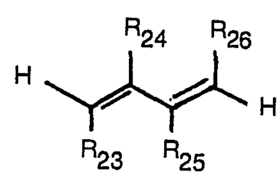

worin

$R_{23}$, $R_{24}$, $R_{25}$ und $R_{26}$ unabhängig voneinander Wasserstoff, $C_1$- bis $C_4$-Alkyl, Vinyl oder Chlor bedeuten, wobei mindestens einer der Reste $R_{23}$ bis $R_{26}$ für Chlor steht,

einsetzt.

6. Verfahren nach Anspruch 1 bis 5, dadurch gekennzeichnet, daß man als halogensubstituiertes Polyen 1,3-Dichlorbutadien, 2,3-Dichlorbutadien, 1-Chlor-butadien oder 2-Chlor-butadien einsetzt.

7. Verfahren nach Anspruch 1 bis 6, dadurch gekennzeichnet, daß man als Lösungsmittel Benzol, Chlorbenzol, 1,2-Dichlorpropan, Tetrachlorkohlenstoff, Propionsäureäthylester oder Cyclohexan einsetzt.

**Revendications**

1. Procédé de production de vinyloxiranes substitués par des halogènes sur le noyau d'oxirane et/ou sur le groupe vinyle à partir de polyènes substitués par des halogènes et d'acides percarboxyliques en solution organique, caractérisé en ce qu'on fait réagir un polyène substitué par un halogène, de formule:

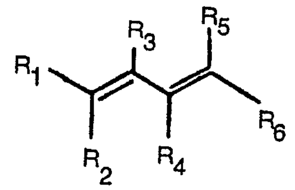

dans laquelle:

$R_1$ et $R_6$ représentent, indépendamment l'un de l'autre, de l'hydrogène, un radical alkyle en $C_1$ à $C_4$, vinyle ou phényle,

$R_2$, $R_3$, $R_4$ et $R_5$ représentent, indépendamment l'un de l'autre, de l'hydrogène un radical alkyle en $C_1$ à $C_4$, vinyle, cycloalkyle en $C_3$ à $C_7$, phényle, du fluor, du chlore ou du brome, et l'un au moins des restes $R_2$ à $R_5$ désigne du fluor, du chlore ou du brome, $R_1$ pouvant former avec $R_2$ ou $R_1$ avec $R_3$ ou $R_1$ avec $R_4$ ou $R_1$ avec $R_5$ ou $R_3$ avec $R_4$, un noyau carbocyclique,

avec une solution d'un acide percarboxylique dans un solvant organique dans un rapport molaire du polyène substitué par un halogène à l'acide percarboxylique de 1,0:1,0 à 10 ou de 1,0 à 10:1,0 et à une température de −20 à +100°C, l'acide percarboxylique contenant jusqu'à 5% en poids d'eau, jusqu'à 2% en poids de peroxyde d'hydrogène et la solution d'acide percarboxylique contenant moins de 50 parties par million d'acide minéral.

2. Procédé suivant la revendication 1, caractérisé en ce qu'on utilise comme polyène substitué par un halogène un polyène de formule:

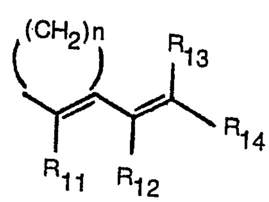

dans laquelle:

$R_{11}$, $R_{12}$ et $R_{13}$ représentent, indépendamment l'un de l'autre, de l'hydrogène, un radical alkyle en $C_1$ à $C_4$, vinyle, cycloalkyle en $C_5$ à $C_7$, phényle, du fluor, du chlore ou du brome, l'un au moins des restes $R_{11}$ à $R_{13}$ représentant du fluor, du chlore ou du brome et

$R_{14}$ désigne de l'hydrogène, un radical alkyle en $C_1$ à $C_4$, vinyle ou phényle,

*n* est un nombre entier de 2 à 10.

3. Procédé suivant les revendications 1 et 2, caractérisé en ce qu'on utilise comme polyène substitué par un halogène un polyène de formule:

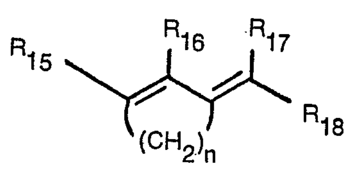

dans laquelle:

$R_{15}$, $R_{16}$ et $R_{17}$ représentent, indépendamment l'un de l'autre, de l'hydrogène, un radical alkyle en $C_1$ à $C_4$, vinyle, cycloalkyle en $C_3$ à $C_7$, phényle, du fluor, du chlore ou du brome, l'un au moins des restes $R_{15}$ à $R_{17}$ étant du fluor, du chlore ou du brome, et

$R_{18}$ est de l'hydrogène ou un radical alkyle en $C_1$ à $C_4$, vinyle ou phényle, et

*n* est un nombre entier de 1 à 9.

4. Procédé suivant les revendications 1 à 3, caractérisé en ce qu'on utilise comme polyène substitué par un halogène, un polyène de formule:

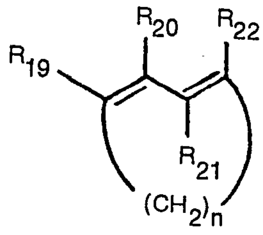

dans laquelle:

$R_{19}$, $R_{20}$, $R_{21}$ et $R_{22}$ représentent, indépendamment l'un de l'autre, de l'hydrogène, un radical alkyle en $C_1$ à $C_4$, vinyle, phényle, cycloalkyle en $C_5$ à $C_7$, du fluor, du chlore ou du brome, l'un au moins des restes $R_{19}$ à $R_{22}$ étant du fluor, du chlore ou du brome.

5. Procédé suivant les revendications 1 à 4, caractérisé en ce qu'on utilise comme polyène substitué par un halogène un polyène de formule:

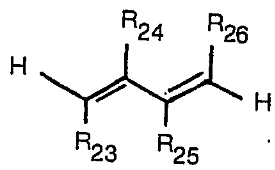

dans laquelle:

$R_{23}$, $R_{24}$, $R_{25}$ et $R_{26}$ représentent, indépendamment l'un de l'autre, de l'hydrogène, un radical alkyle en $C_1$ à $C_4$, un radical vinyle ou du chlore, l'un au moins des restes $R_{23}$ à $R_{26}$ représentant du chlore.

6. Procédé suivant les revendications 1 à 5, caractérisé en ce qu'on utilise comme polyène substitué par un halogène, le 1,3-dichlorobutadiène, le 2,3-dichlorobutadiène, le 1-chlorobutadiène ou le 2-chlorobutadiène.

7. Procédé suivant les revendications 1 à 6, caractérisé en ce qu'on utilise, comme solvant, du benzène, du chlorobenzène, du 1,2-dichloropropane, du tétrachlorure de carbone, du propionate d'éthyle ou du cyclohexane.

**Claims**

1. Process for the preparation of vinyloxiranes which are halogen-substituted on the oxirane ring and/or on the vinyl group from halogen-substituted polyenes and percarboxylic acids in organic solution, characterised in that a halogen-substituted polyene of the formula

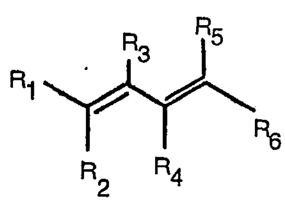

wherein

$R^1$ and $R^6$ independently of one another denote hydrogen, $C_1$- to $C_4$-alkyl, vinyl or phenyl and $R_2$, $R_3$, $R_4$ and $R_5$ independently of one another represent hydrogen, $C_1$- to $C_4$-alkyl, vinyl, $C_3$- to $C_7$-cycloalkyl, phenyl, fluorine, chlorine or bromine, at least one of the radicals $R_2$ to $R_5$ denoting fluorine, chlorine or bromine,

and wherein

$R_1$ and $R_2$, or $R_1$ and $R_3$, or $R_1$ and $R_4$, or $R_1$ and $R_5$, or $R_3$ and $R_4$ can form a carbocyclic ring, is reacted with a solution of a percarboxylic acid in an organic solvent at a molar ratio of halogen-substituted polyene to percarboxylic acid of 1.0:1.0 to 10 or 1.0 to 10:1.0 and at a temperature of −20°C to +100°C, the percarboxylic acid containing up to 5% by weight of water and up to 2% by weight of hydrogen peroxide and the percarboxylic acid solution containing less than 50 ppm of mineral acid.

2. Process according to Claim 1, characterised in that a polyene of the formula

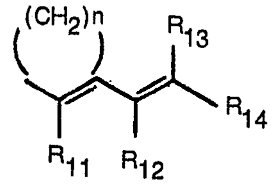

wherein

$R_{11}$, $R_{12}$ and $R_{13}$ independently of one another represent hydrogen, $C_1$- to $C_4$-alkyl, vinyl, $C_5$- to $C_7$ cycloalkyl, phenyl, fluorine, chlorine or bromine, at least one of the radicals $R_{11}$ to $R_{13}$ denoting fluorine, chlorine or bromine, $R_{14}$ denotes hydrogen, $C_1$- to $C_4$-alkyl, vinyl or phenyl and

n represents an integer from 2 to 10,

is employed as the halogen-substituted polyene.

3. Process according to Claim 1 and 2, characterised in that a polyene of the formula

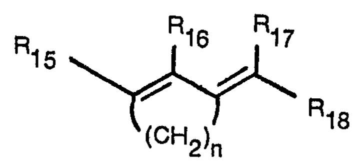

wherein

$R_{15}$, $R_{16}$ and $R_{17}$ independently of one another represent hydrogen, $C_1$- to $C_4$-alkyl, vinyl, $C_3$- to $C_7$-cycloalkyl, phenyl, fluorine, chlorine or bromine, at least one of the radicals $R_{15}$ to $R_{17}$ denoting fluorine, chlorine or bromine,

$R_{18}$ denotes hydrogen, $C_1$- to $C_4$-alkyl, vinyl or phenyl and

n represents an integer from 1 to 9,

is employed as the halogen-substituted polyene.

4. Process according to Claim 1 to 3, characterised in that a polyene of the formula

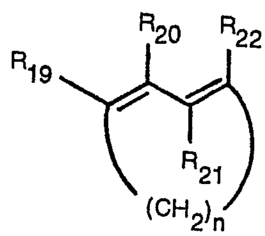

wherein

$R_{19}$, $R_{20}$, $R_{21}$ and $R_{22}$ independently of one another denote hydrogen, $C_1$- to $C_4$-alkyl, vinyl, phenyl, $C_5$- to $C_7$-cycloalkyl, fluorine, chlorine or bromine, at least one of the radicals $R_{19}$ to $R_{22}$ representing fluorine, chlorine or bromine,

is employed as the halogen-substituted polyene.

5. Process according to Claim 1 to 4, characterised in that a polyene of the formula

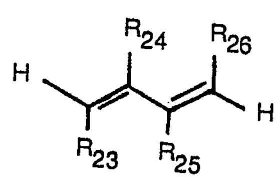

wherein

$R_{23}$, $R_{24}$, $R_{25}$ and $R_{26}$ independently of one another denote hydrogen, $C_1$- to $C_4$-alkyl, vinyl or chlorine, at least one of the radicals $R_{23}$ to $R_{26}$ representing chlorine,

is employed as the halogen-substituted polyene.

6. Process according to Claim 1 to 5, characterised in that 1,3-dichlorobutadiene, 2,3-dichlorobutadiene, 1-chlorobutadiene or 2-chlorobutadiene is employed as the halogen-substituted polyene.

7. Process according to Claim 1 to 6, characterised in that benzene, chlorobenzene, 1,2-dichloropropane, carbon tetrachloride, propionic acid ethyl ester or cyclohexane is employed as the solvent.